(19) Europäisches Patentamt
European Patent Office
Office européen des brevets

(11) Veröffentlichungsnummer: **0 019 189**
**B1**

(12) # EUROPÄISCHE PATENTSCHRIFT

(45) Veröffentlichungstag der Patentschrift: **28.07.82**

(51) Int. Cl.³: **A 01 N 43/64**

(21) Anmeldenummer: **80102450.6**

(22) Anmeldetag: **06.05.80**

(54) **Fungizide Mittel, Verfahren zu ihrer Herstellung sowie ihre Verwendung als Fungizide.**

(30) Priorität: **19.05.79 DE 2920374**

(43) Veröffentlichungstag der Anmeldung: **26.11.80 Patentblatt 80/24**

(45) Bekanntmachung des Hinweises auf die Patenterteilung: **28.07.82 Patentblatt 82/30**

(84) Benannte Vertragsstaaten: **AT BE CH DE FR GB IT LI NL SE**

(56) Entgegenhaltungen: **FR-A-2 333 793**

(73) Patentinhaber: **BAYER AG, Zentralbereich Patente, Marken und Lizenzen, D-5090 Leverkusen 1, Bayerwerk (DE)**

(72) Erfinder: **Regel, Erik, Ing.grad, Bergerheide 72a, D-5600 Wuppertal 1 (DE)**
Erfinder: **Büchel, Karl Heinz, Prof.Dr., Bergerheide 62, D-5600 Wuppertal 1 (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeisterstrasse 5, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 189 B1

# Fungizide Mittel, Verfahren zu ihrer Herstellung, sowie ihre Verwendung als Fungizide

Die vorliegende Erfindung betrifft die Verwendung von Hydroxyalkyltriazolen als Fungizide.

Es ist bereits bekannt geworden, dass im Phenylteil substituierte 1-Phenyl-2-triazolyl-1-äthanole, gute fungizide Eigenschaften besitzen (vgl. DE-OS Nr. 2431407 [Le A 15735]).

Deren Wirksamkeit ist jedoch, insbesondere bei niedrigen Aufwandmengen und -konzentrationen, nicht immer ganz befriedigend.

Weiterhin sind fungizid wirksame 1,3-Diphenyl-2-triazolylpropan-1-ole und 1-Phenyl-2-triazolylhexan-1-ole bekannt geworden, jedoch ist deren Wirksungsspektrum nicht immer voll befriedigend (vgl. FR-PS Nr. 2333799).

Es wurde gefunden, dass die Hydroxyalkyltriazole der allgemeinen Formel

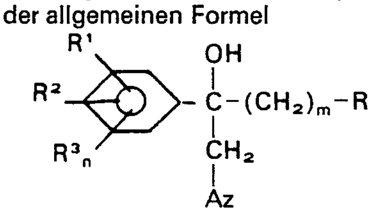

(I)

in welcher

| | |
|---|---|
| Az | für 1,2,4-Triazol-1-yl oder -4-yl steht, |
| R | für gegebenenfalls durch Halogen und/oder Alkyl mit 1 bis 4 Kohlenstoffatomen substituiertes Phenyl, Naphthyl oder Tetrahydronaphthyl steht, |
| $R^1$ | für gegebenenfalls durch Halogen, Alkyl und/oder Alkenyl mit jeweils bis zu 4 Kohlenstoffatomen substituiertes Phenyl, Cyclopentyl oder Cyclohexyl steht und |
| $R^2$ | für Wasserstoff steht oder |
| $R^1$ und $R^2$ | gemeinsam in o-Stellung zueinander für eine Methylenbrücke mit 3 bis 5 Methylengruppen stehen, |
| $R^3$ | für Halogen steht, |
| n | für 0, 1, 2 oder 3 steht und |
| m | für 0 oder 1 steht, |

und deren physiologisch verträglichen Säureadditions-Salze gute fungizide Eigenschaften aufweisen.

Überraschenderweise zeigen die erfindungsgemäss verwendbaren Hydroxyalkyltriazole der Formel (I) eine bessere fungizide Wirksamkeit als die aus dem Stand der Technik bekannten substituierten 1-Phenyl-2-triazolyl-1-äthanole, welche chemisch und wirkungsmässig naheliegende Verbindungen sind. Die erfindungsgemäss verwendbaren Stoffe stellen somit eine Bereicherung der Technik dar.

Bevorzugt sind diejenigen Verbindungen der Formel (I), in denen Az die oben angegebene Bedeutung hat, R für Phenyl, das gegebenenfalls einfach oder zweifach, gleich oder verschieden durch Chlor, Fluor oder Methyl substituiert ist, für Naphthyl und Tetrahydronaphthyl steht; $R^1$ für Phenyl, Cyclopentyl oder Cyclohexyl steht, diese Reste können gegebenenfalls einfach oder zweifach durch Chlor, Brom, Fluor, Methyl, Äthyl, Isopropyl oder Isopropenyl substituiert sein; und $R^2$ für Wasserstoff steht, oder $R^1$ und $R^2$ gemeinsam in o-Stellung zueinander für eine Tri-, Tetra- oder Pentamethylenbrücke stehen; $R^3$ für Chlor, Fluor oder Methyl steht, n für 0 oder 1 steht und m für 0 oder 1 steht.

Im einzelnen seien ausser den bei den Herstellungsbeispielen genannten Verbindungen die folgenden Verbindungen der allgemeinen Formel (I) genannt (wobei Az jeweils für 1,2,4-Triazol-1-yl oder 4-yl steht):

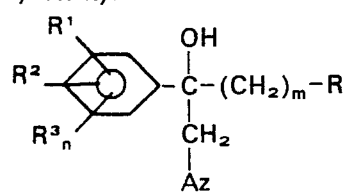

(I)

| R | $R^1$ | $R^2$ | $R^3_n$ | m |
|---|---|---|---|---|
| 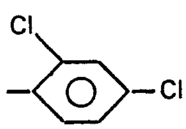 | 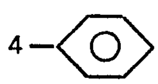 | H | — | 0 |
| 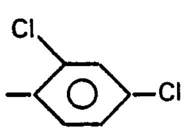 | 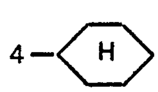 | H | — | 0 |
| 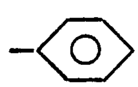 | 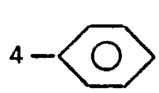 | H | — | 0 |
| 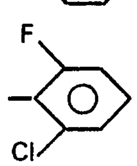 | 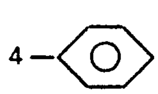 | H | — | 0 |

| R | $R^1$ | $R^2$ | $R^3{}_n$ | m |
|---|---|---|---|---|
| 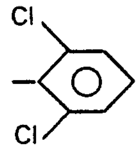 | 4 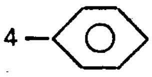 | H | — | 0 |
| 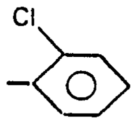 | 4 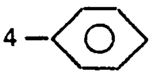 | H | — | 0 |
| 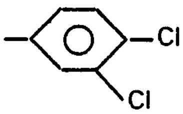 | 4 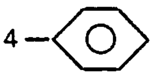 | H | — | 0 |
| 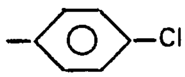 | $3,4-(CH_2)_3-$ | | — | 0 |
| 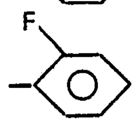 | 4 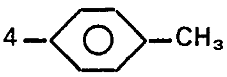 | H | — | 0 |
| 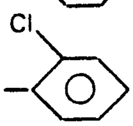 | 4 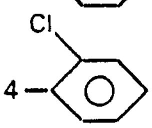 | H | — | 0 |
| 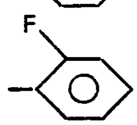 | 4 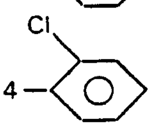 | H | — | 0 |
| 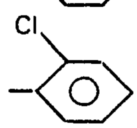 | 4 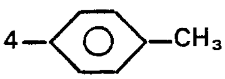 | H | — | 0 |
| 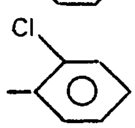 | 4 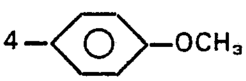 | H | — | 0 |
| 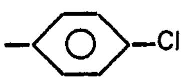 | 4 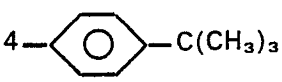 | H | — | 0 |
| 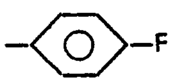 | 4 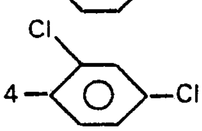 | H | — | 0 |
| 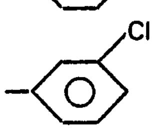 | 4 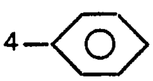 | H | — | 0 |
| 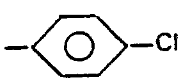 | $3,4-(CH_2)_4-$ | | — | 0 |
| 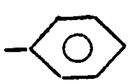 | 4 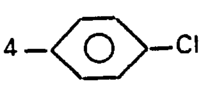 | H | — | 0 |
| 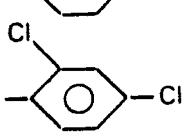 | 4 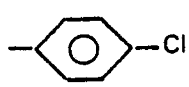 | H | — | 0 |
| 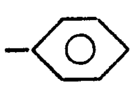 | 4 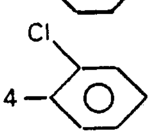 | H | — | 0 |

| R | $R^1$ | $R^2$ | $R^3_n$ | m |
|---|---|---|---|---|
| 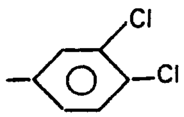 | 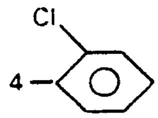 | H | — | 0 |
| 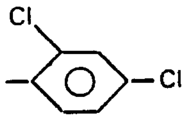 | 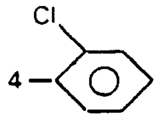 | H | — | 0 |
| 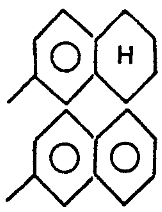 | 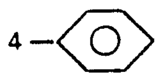 | H | — | 0 |
| | 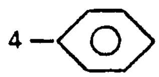 | H | — | 0 |
| 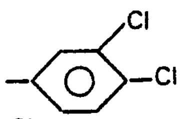 | 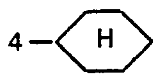 | H | — | 0 |
| 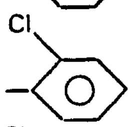 | $3,4-(CH_2)_4-$ | | — | 0 |
| 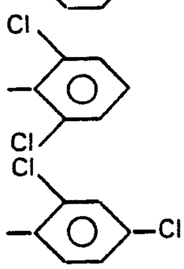 | $3,4-(CH_2)_4-$ | | — | 0 |
| | 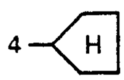 | H | — | 0 |
| 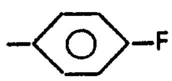 | 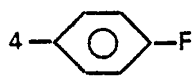 | H | — | 0 |
| 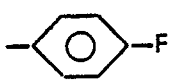 | 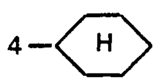 | H | — | 0 |
| 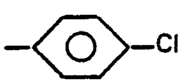 | 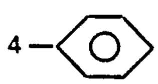 | H | — | 0 |
| 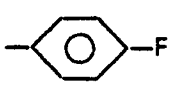 | 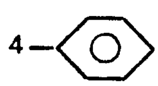 | H | — | 0 |
| 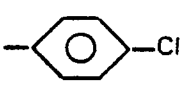 | 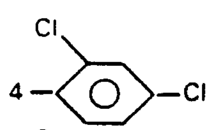 | H | — | 0 |
| 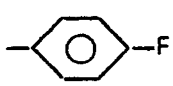 | 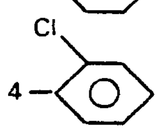 | H | — | 0 |
| 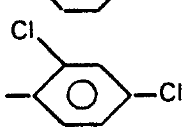 | 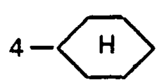 | H | — | 1 |
| 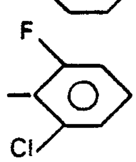 | 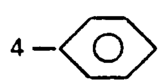 | H | — | 1 |

| R | $R^1$ | $R^2$ | $R^3{}_n$ | m |
|---|---|---|---|---|
| 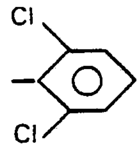 | 4 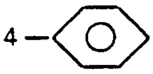 | H | — | 1 |
| 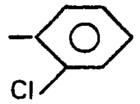 | 4 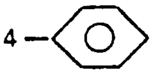 | H | — | 1 |
| 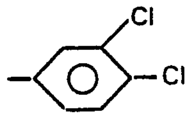 | 4 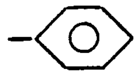 | H | — | 1 |
| 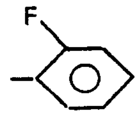 | 4 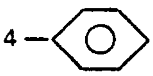 | H | — | 1 |
| 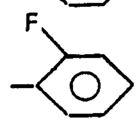 | 4 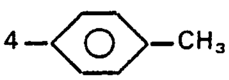 | H | — | 1 |
| 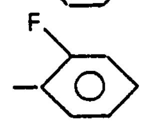 | 4 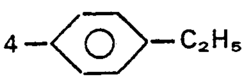 | H | — | 1 |
| 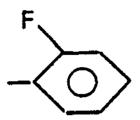 | 4 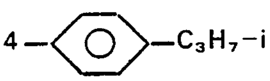 | H | — | 1 |
| 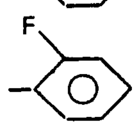 | 4 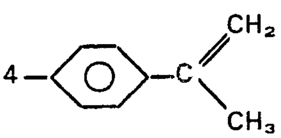 | H | — | 1 |
| 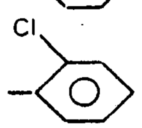 | 4 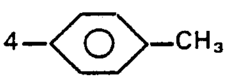 | H | — | 1 |
| 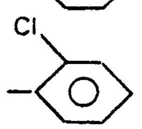 | 4 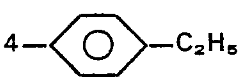 | H | — | 1 |
| 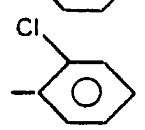 | 4 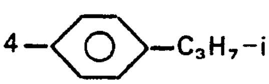 | H | — | 1 |
| 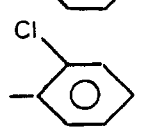 | 4 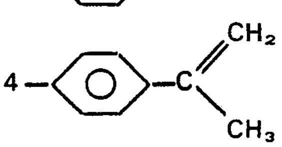 | H | — | 1 |
| 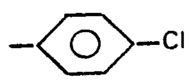 | 4 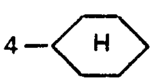 | H | — | 1 |
| 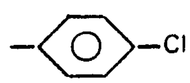 | 3,4-$(CH_2)_3$- | | — | 1 |
| 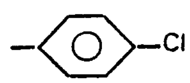 | 3,4-$(CH_2)_4$- | | — | 1 |
| 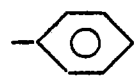 | 4 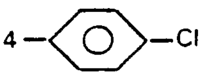 | H | — | 1 |

| R | $R^1$ | $R^2$ | $R^3_n$ | m |
|---|---|---|---|---|
| 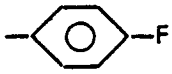 | 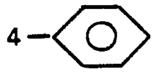 | H | — | 1 |
| 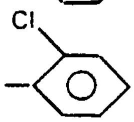 | 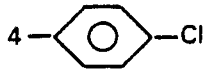 | H | — | 1 |
| 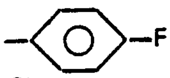 | 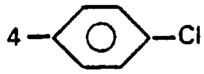 | H | — | 1 |
| 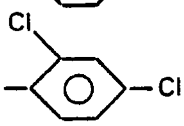 | 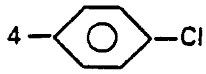 | H | — | 1 |
| 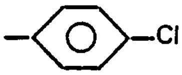 | 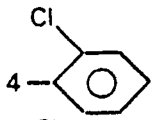 | H | — | 1 |
| 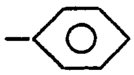 | 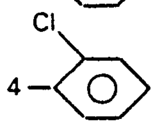 | H | — | 1 |
| 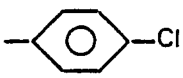 | 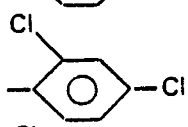 | H | — | 1 |
| 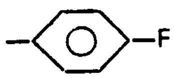 | 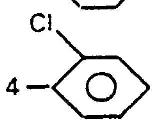 | H | — | 1 |
| 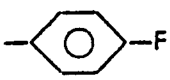 | 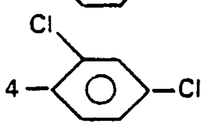 | H | — | 1 |
| 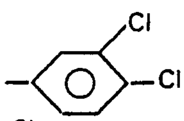 | 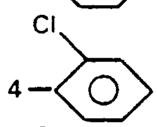 | H | — | 1 |
| 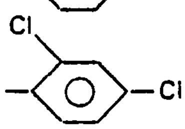 | 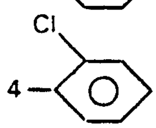 | H | — | 1 |
| 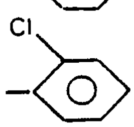 | 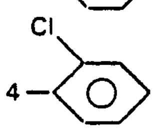 | H | — | 1 |
| 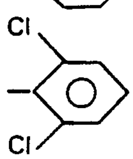 | 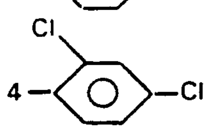 | H | — | 1 |
| 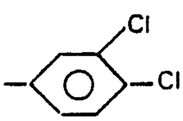 | 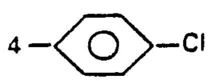 | H | — | 1 |
| 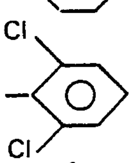 | 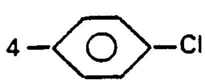 | H | — | 1 |
| 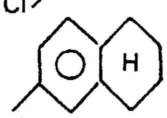 | 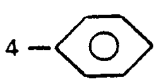 | H | — | 1 |

| R | $R^1$ | $R^2$ | $R^3_n$ | m |
|---|---|---|---|---|
| 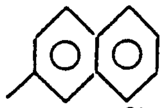 | 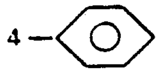 | H | — | 1 |
| 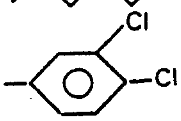 | 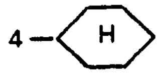 | H | — | 1 |
| 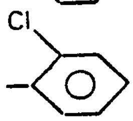 | 3,4-$(CH_2)_4$- | | — | 1 |
| 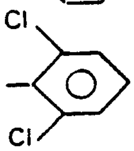 | 3,4-$(CH_2)_3$- | | — | 1 |
| 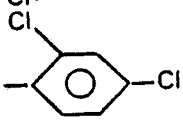 | 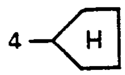 | H | — | 1 |
| 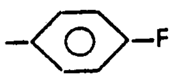 | 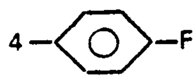 | H | — | 1 |
| 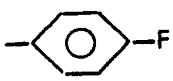 | 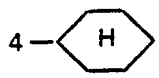 | H | — | 1 |

Die erfindungsgemäss zu verwendenden Wirkstoffe und deren Säureadditionssalze sind noch nicht beschrieben. Die Stoffe und deren Herstellung sind jedoch Gegenstand von eigenen älteren Anmeldungen (vgl. die deutschen Offenlegungsschriften Nr. 2851086 [Le A 19 272] vom 25.11.1978, und Nr. 2851116 [Le A 19 273] vom 25.11.1978); und können nach den dort beschriebenen Verfahren hergestellt werden, indem man

a) Triazolylmethylphenylketone der Formel

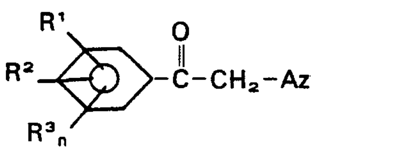 (II)

in welcher

Az, $R^1$, $R^2$, $R^3$ und n die oben angegebene Bedeutung haben,

mit einer Grignard-Verbindung der Formel

$$R-(CH_2)_m-Mg-X \quad (III)$$

in welcher

R und m die oben angegebene Bedeutung haben und

X für Halogen, insbesondere Chlor oder Brom steht,

in Gegenwart eines für eine Grignard-Reaktion üblichen Lösungsmittels, wie vorzugsweise Äther, bei Temperaturen zwischen etwa 30 bis etwa 80° C umsetzt, oder

b) 1-Halogenalkylalkohole der Formel

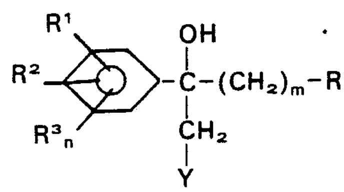 (IV)

in welcher

R, $R^1$, $R^2$, $R^3$, n und m die oben angegebene Bedeutung haben und

Y für Halogen, insbesondere Chlor oder Brom, steht,

mit Triazol, gegebenenfalls in Gegenwart eines Säurebindemittels, wie z.B. einem Überschuss an Triazol, und gegebenenfalls in Gegenwart eines inerten organischen Lösungsmittels, wie z.B. Acetonitril, bei Temperaturen zwischen 30 bis 200° C, vorzugsweise bei der Siedetemperatur des Lösungsmittels, umsetzt. In manchen Fällen erweist es sich als vorteilhaft, die Triazole in Form ihrer Alkalisalze, wie das Natrium- oder Kaliumsalz, einzusetzen.

Die erfindungsgemäss zu verwendenden Wirkstoffe, in denen der Index m für O steht, können auch nach einem weiteren Verfahren erhalten werden, welches jedoch ebenfalls noch nicht beschrieben ist (vgl. die deutsche Offenlegungsschrift Nr. 2912288. [Le A 19 527] vom 28.3.1979), indem man

c) Oxirane der Formel

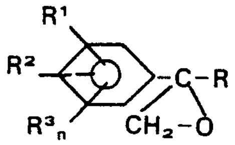

(V)

in welcher

R, $R^1$, $R^2$, $R^3$ und n die oben angegebene Bedeutung haben,

mit Triazol in Gegenwart eines Alkalialkoholats, wie z.B. Natriummethylat und in Gegenwart eines inerten organischen Lösungsmittels, wie z.B. Dimethylformamid, bei Temperaturen zwischen 30 und 100° C umsetzt.

Die Triazolylmethylphenylketone der Formel (II) sind noch nicht bekannt. Sie lassen sich jedoch in allgemein üblicher und bekannter Weise herstellen, indem man entsprechende Phenacylhalogenide (das sind α-Halogenacetophenonderivate) mit Triazol in Gegenwart eines Verdünnungsmittels, wie beispielsweise Dimethylformamid, und in Gegenwart eines säurebindenden Mittels, wie insbesondere einem Überschuss an Triazol, bei Temperaturen zwischen 20 und 80° C umsetzt.

Die Grignard-Verbindungen der Formel (III) sind allgemein bekannte Verbindungen der organischen Chemie.

Die 1-Halogenalkylalkohole der Formel (IV) sind noch nicht bekannt. Sie lassen sich jedoch in allgemein üblicher und bekannter Weise herstellen, indem man entsprechende Phenacylhalogenide mit Grignard-Verbindungen der Formel (III) nach Verfahrensvariante (a) umsetzt.

Die Oxirane der Formel (V) sind noch nicht bekannt. Auch sie sind Gegenstand der erwähnten vorgängigen eigenen Anmeldung (vgl. die deutsche Offenlegungsschrift Nr. 2912288 [Le A 19 527] vom 28.3.1979) und können erhalten werden, indem man entsprechende Ketone entweder mit Dimethyloxosulfoniummethylid in an sich bekannter Weise in Gegenwart eines Verdünnungsmittels, wie beispielsweise Dimethylsulfoxid, bei Temperaturen zwischen 20 und 80° C umsetzt; oder mit Trimethylsulfoniummethylsulfat in an sich bekannter Weise in Gegenwart eines Zweiphasensystems und gegebenenfalls in Gegenwart eines Phasentransferkatalysators bei Temperaturen zwischen 0 und 100° C umsetzt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Podosphaera-, Uromyces- und Erysiphearten eingesetzt werden, wie gegen den Erreger des echten Apfelmehltaus (Podosphaera leucotricha), den Erreger des Bohnenrostes (Uromyces phaseoli) und gegen den Erreger des echten Gurkenmehltaus (Erysiphe cichoacearum). Gute Erfolge werden auch bei der Bekämpfung von Getreidekrankheiten, wie Getreidemehltau und Getreiderost, erzielt.

In bestimmten Aufwandmengen zeigen die erfindungsgemässen Wirkstoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Ferroxyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 Gew.%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstruktur-Verbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.%, vorzugsweise zwischen 0,5 und 0,001 Gew.%.

Bei der Saatgutbehandlung werden im allgemeinen Wirkstoffmengen von 0,001 bis 50 g/kg Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,02 Gew.%, am Wirkungsort erforderlich.

In den nachfolgenden Beispielen werden die nachstehend angegebenen Verbindungen als Vergleichssubstanzen eingesetzt:

(A) 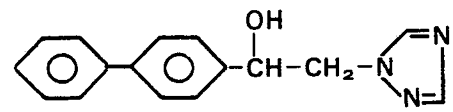

(B) 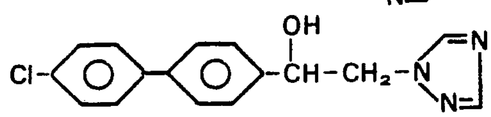

(C) 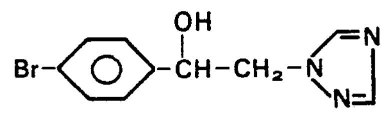

(D) 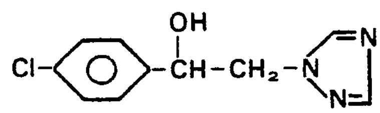

(E) 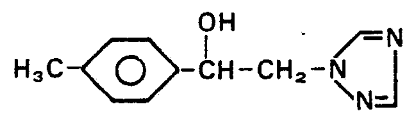

(F) 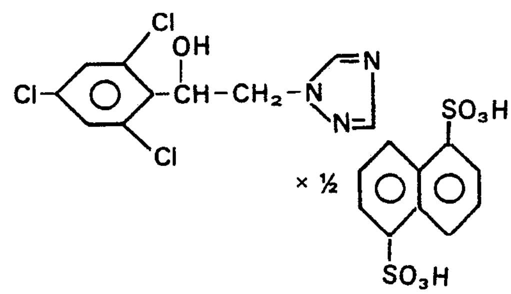

*Beispiel A*

Podosphaera-Test (Apfel)/protektiv
Lösungsmittel: 4,7 Gewichtsteile Aceton
Emulgator: 0,3 Gewichtsteile Alkylarylpolyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20° C und einer relativen Luftfeuchtigkeit

von 70% im Gewächshaus. Anschliessend werden sie durch Bestäuben mit Konidien des Apfelmehltauerregers (Podosphaera leucotricha) inokuliert und in ein Gewächshaus mit einer Temperatur von 21 bis 23° C und einer relativen Luftfeuchtigkeit von 70% gebraucht.

10 d nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

In diesem Test zeigen z.B. folgende Verbindungen eine sehr gute Wirkung, die derjenigen der aus dem Stand der Technik bekannten Verbindungen (A) und (B) deutlich überlegen ist:

Verbindungen gemäss Herstellungsbeispielen 1 und 3.

*Beispiel B*

Erysiphe-Test (Gurken)/Protektiv
Lösungsmittel: 4,7 Gewichtsteile Aceton
Emulgator: 0,3 Gewichtsteile Alkylarylpolyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthalt.

Mit der Spritzflüssigkeit bespritzt man junge Gurkenpflanzen mit etwa drei Laubblättern bis zur Tropfnässe. Die Gurkenpflanzen verbleiben zur Trocknung 24 h im Gewächshaus. Dann werden sie zur Inokulation mit Konidien des Pilzes Erysiphe cichoracearum bestäubt. Die Pflanzen werden anschliessend bei 23 bis 24° C und bei einer relativen Luftfeuchtigkeit von ca. 75% im Gewächshaus aufgestellt.

Nach 12 d wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

Bei diesem Test zeigen z.B. die folgenden Verbindungen eine sehr gute Wirkung, die derjenigen der aus dem Stand der Technik bekannten Verbindungen (C) und (D) überlegen ist:

Verbindungen gemäss Herstellungsbeispielen: 1, 7, 10, 12, 14 und 5.

*Beispiel C*

Uromyces-Test (Bohnenrost)/Protektiv
Lösungsmittel: 4,7 Gewichtsteile Aceton
Emulgator: 0,3 Gewichtsteile Alkylarylpropylglykoläther
Wasser: 95,0 Gewichtsteile

Man vermischt die für die gewünschte Wirkstoffkonzentration in der Spritzflüssigkeit notwendige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man die jungen Bohnenpflanzen, die sich im 2-Blattstadium befinden, bis zur Tropfnässe. Die Pflanzen verbleiben zum Abtrocknen 24 h bei 20 bis 22° C und einer relativen Luftfeuchtigkeit von 70% im Gewächshaus. Anschliessend werden sie mit einer wässrigen Uredosporensuspension des Bohnenrosterregers (Uromyces phaseoli) inokuliert und 24 h lang in einer dunklen Feuchtkammer bei 20 bis 22° C und 100% relativer Luftfeuchtigkeit inkubiert.

Die Pflanzen werden dann unter intensiver Belichtung für 9 d bei 20 bis 22° C und einer relativen Luftfeuchtigkeit von 70 bis 80% im Gewächshaus aufgestellt.

10 d nach der Inokulation wird der Befall der Pflanzen bestimmt. Die erhaltenen Boniturwerte werden in Prozent Befall umgerechnet. 0% bedeutet keinen Befall, 100% bedeutet, dass die Pflanzen vollständig befallen sind.

In diesem Test zeigen z.B. folgende Verbindungen eine sehr gute Wirkung, die derjenigen der aus dem Stand der Technik bekannten Verbindung(en) (E) deutlich überlegen ist:

Verbindungen gemäss Herstellungsbeispielen 12, 14 und 5.

*Beispiel D*

Sprossbehandlungstest/Getreidemehltau/Protektiv (blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator (Alkylarylpolyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 d Verweilzeit der Pflanzen bei einer Temperatur von 21 bis 22° C und einer Luftfeuchtigkeit von 80 bis 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Bei diesem Test zeigen z.B. die folgenden Verbindungen eine sehr gute Wirkung, die derjenigen der aus dem Stand der Technik bekannten Verbindungen (D) und (F) deutlich überlegen ist:

Verbindungen gemäss Herstellungsbeispielen 1, 3, 7, 10, 12, 14 und 5.

*Beispiel E*

Sprossbehandlungstest/Getreiderost/protektiv (blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator Alkylarylpropylglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 h bei etwa 20° C und einer 100%igen Luftfeuchtigkeit in ein Gewächshaus.

Nach 10 d Verweilzeit der Pflanzen bei einer Temperatur von 20° C und einer Luftfeuchtigkeit von 80 bis 90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Rostbefall ist.

In diesem Test zeigen z.B. die folgenden Verbindungen eine sehr gute Wirkung, die derjenigen der aus dem Stand der Technik bekannten Verbindung (D) deutlich überlegen ist:

Verbindungen gemäss Herstellungsbeispielen 8, 12, 14 und 5.

*Herstellungsbeispiele*

*Beispiel 1 und 2*

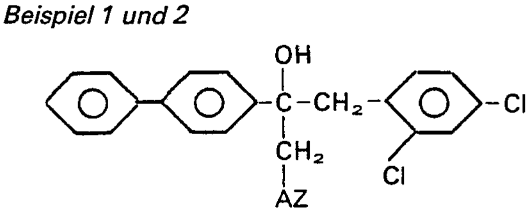

Az = 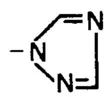 = Beispiel 1

Az = 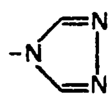 = Beispiel 2

(Verfahren b)

Eine Lösung von 3,5 g (0,065 mol) Natriummethylat in 18 ml Methylalkohol wird mit 7,6 g (0,11 mol) 1,2,4-Triazol versetzt; anschliessend wird eine Lösung von 19,5 g (0,05 mol) 2-(4-Biphenyl)-3-chlor-1-(2,4-dichlorphenyl)propan-2-ol in 38 ml Dimethylformamid zugetropft und 90 min auf 70° C erhitzt. Die Lösungsmittel werden im Vakuum an Rotationsverdampfer entfernt und der Rückstand mit Wasser verrührt. Die resultierenden Kristalle werden mit Diethyläther gewaschen und aus Acetonitril umkristallisiert. Man erhält 2,2 g 2-(4-Biphenylyl)-1-(2,4-dichlorphenyl)-3-(1,2,4-triazol-4-yl)propan-2-ol vom Schmelzpunkt 260° C.

Die Acetonitrillösung (Mutterlauge) wird eingedampft und die resultierenden Kristalle werden mit Diethyläther und Essigester gewaschen. Man erhält 6,5 g 2-(4-Biphenylyl)-1-(2,4-dichlorphenyl)-3-(1,2,4-triazol-1-yl)propan-2-ol vom Schmelzpunkt 124° C.

*Herstellung des Ausgangsproduktes*

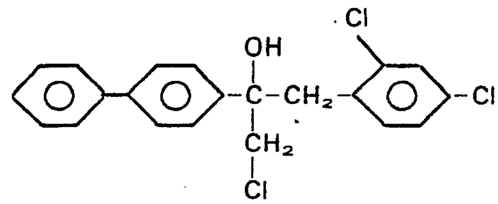

Zu einer Lösung von 0,6 mol 2,4-Dichlorbenzylmagnesiumchlorid, erhalten aus 15,9 g (0,65 mol) Magnesium und 117,3 g (0,6 mol) 2,4-Dichlorbenzylchlorid in 300 ml Äther, werden 69,3 g (0,3 mol) 4-Phenylphenacylchlorid portionsweise zugegeben. Anschliessend wird das Reaktionsgemisch auf wässrige Ammoniumchloridlösung gegossen, die Ätherphase abgetrennt, mit Wasser gewaschen, über Natriumsulfat getrocknet und eingedampft. Das verbleibende Öl wird mit Petroläther extrahiert und die Petrolätherlösung eingedampft. Die Kristalle werden abfiltriert und getrocknet. Man erhält 62 g (53% der Theorie) 2-(4-Biphenylyl)-3-chlor-1-(2,4-dichlorphenyl)-propan-2-ol vom Schmelzpunkt 84° C.

*Beispiele 3 und 4*

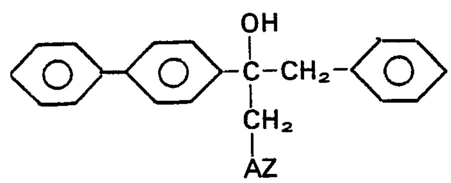

Az = 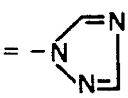 = Beispiel 3

Az = 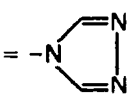 = Beispiel 4

(Verfahren b)

Eine Lösung von 17,9 g (0,33 mol) Natriummethylat in 90 ml Methylalkohol wird mit 38,3 g (0,56 mol) 1,2,4-Triazol versetzt; anschliessend wird eine Lösung von 82 g (0,254 mol) 2-(4-Biphenylyl)-3-chlor-1-phenylpropan-2-ol in 191 ml Dimethylformamid zugetropft und 90 min auf 60° C erhitzt. Die Lösungsmittel werden im Vakuum am Rotationsverdampfer entfernt; der Rückstand wird in Methylenchlorid gelöst und mit Wasser gewaschen. Die Methylenchloridlösung wird über Natriumsulfat getrocknet, filtriert und im Vakuum eingeengt. Das resultierende Öl wird chromatographisch getrennt. Man erhält 13,8 g 2-(4-Biphenylyl)-1-phenyl-3-(1,2,4-triazol-1-yl)propan-2-ol vom Schmelzpunkt 124° C und 2,7 g 2-(4-Biphenylyl)-1-phenyl-3-(1,2,4-triazol-4-yl)propan-2-ol vom Schmelzpunkt 246° C.

*Herstellung des Ausgangsproduktes*

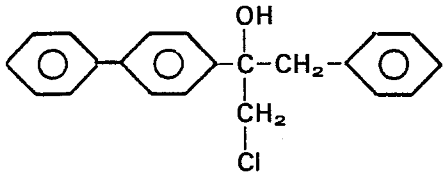

Zu einer Lösung von Benzylmagnesiumchlorid, erhalten aus 24,3 g (1 mol) Magnesium und 115 ml (1 mol) Benzylchlorid in 150 ml Diethyläther, werden 115,3 g (0,5 mol) 4-Phenylphenacylchlorid potionsweise zugegeben. Das Reaktionsgemisch wird 90 min rückfliessend erwärmt und anschliessend auf wässrige Ammoniumchloridlösung gegossen. Die abgetrennte Ätherphase wird mit Wasser gewaschen, über Natriumsulfat getrocknet und eingeengt. Das verbleibende Öl wird durch Verrühren mit Petroläther zur Kristallisation gebracht. Man erhält 50 g (31% der Theorie) 2-(4-Biphenylyl)-3-chlor-1-phenylpropan-2-ol vom Schmelzpunkt 96° C.

*Beispiel 5*

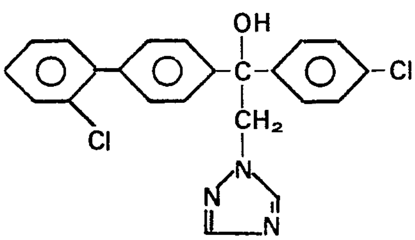

(Verfahren b)

Eine Lösung von 7,02 g (0,13 mol) Natriummethylat in 36 ml Methylalkohol wird mit 14,96 g (0,22 mol) Triazol versetzt. Anschliessend wird eine Lösung von 40 g (0,1 mol) 1-(2'-Chlor-4-biphenylyl)-2-chlor-1-(4-chlorphenyl)äthanol in 75 ml Dimethylformamid zugetropft und 3 h auf 70° C erhitzt. Die Reaktionsmischung wird durch Abdestillieren der Lösungsmittel im Vakuum eingeengt und der Rückstand mit Wasser verrührt. Die verbleibenden Kristalle werden mit Acetonitril gewaschen und umkristallisiert. Man erhält 16,2 g (40% der Theorie) 1-(2'-Chlor-4-biphenylyl)-1-(4-chlorphenyl)-2-(1,2,4-triazol-1-yl)äthanol vom Schmelzpunkt 190° C.

*Herstellung des Ausgangsproduktes*

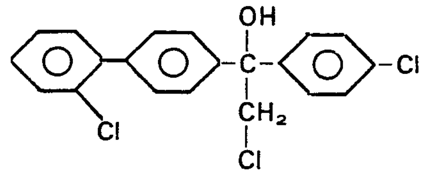

Aus 10,69 g (0,44 mol) Magnesium, 76,6 g (0,4 mol) 4-Bromchlorbenzol und 53 g (0,2 mol) 4-(2-Chlorphenyl)phenacylchlorid, erhält man analog Beispiel 1 75 g 1-(2'-Chlor-4-biphenylyl)-2-chlor-1-(4-chlorphenyl)äthanol.

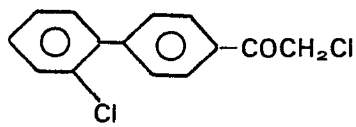

Zu einer Lösung von 377 g (2 mol) 2-Chlorbiphenyl in 160 ml (2 mol) Chloracetylchlorid und 1000 ml Methylenchlorid werden 293,7 g (2,2 mol) Aluminiumchlorid portionsweise eingetragen. Nach 18 h wird das Reaktionsgemisch auf Eis und Salzsäure gegossen. Die organische Phase wird abgetrennt, gewaschen, über Natriumsulfat getrocknet und im Vakuum durch Abdestillieren des Lösungsmittels eingeengt. Das verbleibende Öl wird destillativ gereinigt. Man erhält 478,7 g (90% der Theorie) 4-(2-Chlorphenyl)phenacylchlorid vom Schmelzpunkt 47° C.

Analog den Beispielen 1 bis 5 können die folgenden Verbindungen der allgemeinen Formel

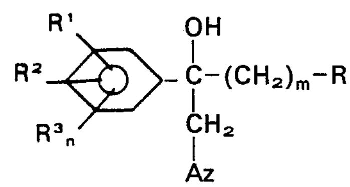 (I)

erhalten werden:

| Bsp. Nr. | R | $R^1$ | $R^2$ | $R^3_n$ | Az | m | Schmelzpunkt (° C) bzw. Brechungsindex |
|---|---|---|---|---|---|---|---|
| 6 | 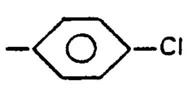 | 4- 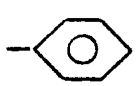 | H | — | 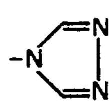 | 1 | 250 |
| 7 | 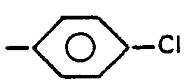 | 4- 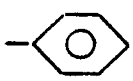 | H | — | 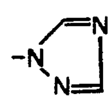 | 1 | 104 |
| 8 | 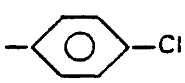 | 4- 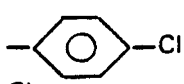 | H | — | 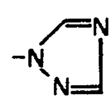 | 1 | 144 |
| 9 | 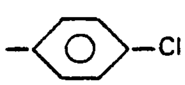 | 4- 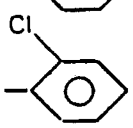 | H | — | 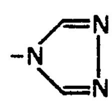 | 1 | 175 |

| Bsp. Nr. | R | R¹ | R² | $R^3_n$ | Az | m | Schmelzpunkt (° C) bzw. Brechungsindex |
|---|---|---|---|---|---|---|---|
| 10 | 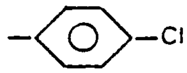 | 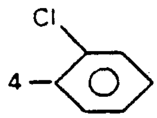 | H | — | 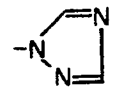 | 1 | $n_D^{20}$: 1,5992 |
| 11 | 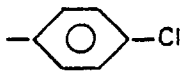 | 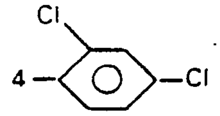 | H | — | 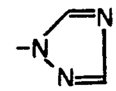 | 1 | 160 |
| 12 | 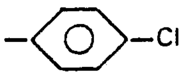 | 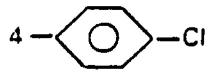 | H | — | 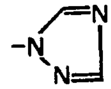 | 0 | 189 |
| 13 | 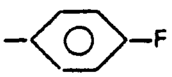 | 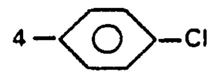 | H | — | 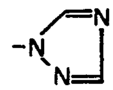 | 0 | 217 |
| 14 | 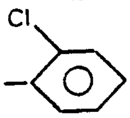 | 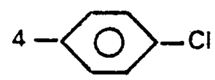 | H | — | 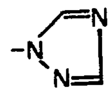 | 0 | 144 |
| 15 | 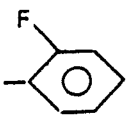 | 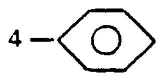 | H | — | 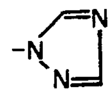 | 0 | 164 |
| 16 | 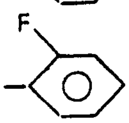 | 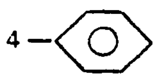 | H | — | 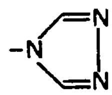 | 0 | 170 |
| 17 | 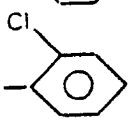 | 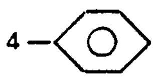 | H | — | 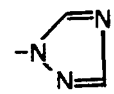 | 0 | 160 |
| 18 | 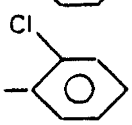 | 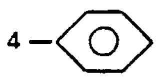 | H | — | 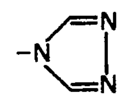 | 0 | 220 |

## Patentansprüche

1. Fungizide Mittel, gekennzeichnet durch einen Gehalt an mindestens einem Hydroxyalkyltriazol der allgemeinen Formel

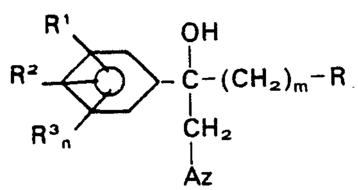

(I)

in welcher

Az für 1,2,4-Triazol-1-yl oder -4-yl steht,
R für gegebenenfalls durch Halogen und/oder Alkyl mit 1 bis 4 Kohlenstoffatomen substituiertes Phenyl, Naphthyl oder Tetrahydronaphthyl steht,
R¹ für gegebenenfalls durch Halogen, Alkyl und/oder Alkenyl mit jeweils bis zu 4 Kohlenstoffatomen substituiertes Phenyl, Cyclopentyl oder Cyclohexyl steht und
R² für Wasserstoff steht oder
R¹ und R² gemeinsam in o-Stellung zueinander für eine Methylenbrücke mit 3 bis 5 Methylengruppen stehen,
R³ für Halogen steht,
n für 0, 1, 2 oder 3 steht und
m für 0 oder 1 steht,

oder deren physiologisch verträglichen Säureadditionssalzen.

2. Verwendung von Hydroxyalkyltriazolen der Formel I zur Bekämpfung von Pilzen.

3. Verfahren zur Herstellung von fungiziden Mitteln, dadurch gekennzeichnet, dass man Hydroxyalkyltriazole der Formel I mit Streckmitteln und/oder oberflächenaktiven Mitteln vermischt.

## Claims

1. Fungicidal agents, characterized in that they contain at least one hydroxyalkyltriazole of the general formula

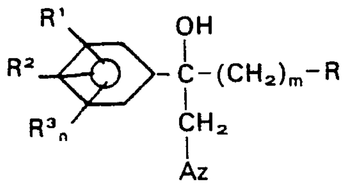

(I)

in which

Az represents 1,2,4-triazol-1-yl or 1,2,4-triazol-4-yl,

R represents phenyl, naphthyl or tetrahydronaphthyl optionally substituted by halogen and/or alkyl with 1 to 4 carbon atoms,

$R^1$ represents phenyl, cyclopentyl or cyclohexyl optionally substituted by halogen, alkyl and/or alkenyl with in each case up to 4 carbon atoms and

$R^2$ represents hydrogen or

$R^1$ and $R^2$ together, in the o-position relative to one another, represent a methylene bridge with 3 to 5 methylene groups,

$R^3$ represents halogen,

n represents 0, 1, 2 or 3 and

m represents 0 or 1,

or physiologically acceptable acid addition salts thereof.

2. Use of hydroxyalkyltriazoles of the formula I for combating fungi.

3. Process for the preparation of fungicidal agents, characterized in that hydroxyalkyltriazoles of the formula I are mixed with extenders and/or surface-active agents.

## Revendications

1. Produits fongicides, caractérisés en ce qu'ils contiennent au moins un hydroxyalkyltriazole de formule

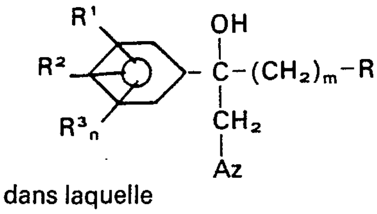

(I)

dans laquelle

Az représente un groupe 1,2,4-triazole-1-yle ou 4-yle,

R représente un groupe phényle, naphtyle ou tétrahydronaphtyle éventuellement substitué par des halogènes et/ou des groupes alkyle en $C_1$-$C_4$,

$R^1$ représente un groupe phényle, cyclopentyle ou cyclohexyle éventuellement substitué par des halogènes, des groupes alkyle et/ou alcényle contenant chacun jusqu'à 4 atomes de carbone, et

$R^2$ représente l'hydrogène ou bien

$R^1$ et $R^2$ forment ensemble, en position ortho, l'un par rapport à l'autre, un pont méthyléné de 3 à 5 groupes méthylène,

$R^3$ représente un halogène,

n est égal à 0, 1, 2 ou 3, et

m est égal à 0 ou 1,

ou leurs sels formés par addition avec des acides tolérés par l'organisme.

2. Utilisation des hydroxyalkyltriazoles de formule I pour combattre les mycètes.

3. Procédé de préparation de produits fongicides, caractérisé en ce que l'on mélange les hydroxyalkyltriazoles de formule I avec des diluants et/ou des agents tensio-actifs.